# FASCICULE DE BREVET EUROPEEN

(11) **EP 3 781 070 B1**
(45) Date de publication et mention de la délivrance du brevet: **30.10.2024**
(21) Numéro de dépôt: 19721205.3
(22) Date de dépôt: 12.04.2019
(51) Int. Cl.: A61B 50/33, A61L 2/07

(54) **ENSEMBLE COMPRENANT EMBALLAGE, PLATEAU ET POCHE À USAGE MÉDICAL, TUBULURES ET ACCESSOIRES, DESTINÉ À UNE STÉRILISATION**
KOMBINATION AUS UMVERPACKUNG, SCHALE, BEUTEL FÜR MEDIZINISCHE VERWENDUNG, SCHLÄUCHE UND ZUBEHÖR ZUR STERILISATION
KIT COMPRISING AN ENVELOPE, A TRAY, BAG FOR MEDICAL USE, TUBES AND ACCESSORIES, INTENDED FOR STERILISATION

(30) Priorité: 16.04.2018 FR 1853309
(43) Date de publication de la demande: 24.02.2021
(73) Titulaire: Technoflex, 64210 Bidart (FR)
(72) Inventeur: ANGOT, Maxime, 64210 Ahetze (FR)
(74) Mandataire: Regimbeau
(86) Numéro de dépôt international: PCT/EP2019/059429
(87) Numéro de publication internationale: WO 2019/201776

(56) Documents cités:
- WO-A1-2014/002938
- FR-A1- 2 699 146
- GB-A- 2 188 549
- US-A1- 2016 015 456
- US-A1- 2017 202 699
- US-A1- 2017 290 634
- US-B2- 10 111 710

## Description

### Domaine de l'invention

La présente invention se rapporte au domaine du conditionnement de systèmes creux et souples stérilisés à la vapeur.

La présente invention concerne plus particulièrement un ensemble suivant l'objet de la revendication indépendante.

### Etat de la technique

Les besoins suivants existent dans l'état de la technique :
- A l'étape d'emballage / conditionnement, à la fois :
   ∘ Permettre la mise en position ergonomique et répétable du système (poche, tubulures et accessoires) dans un sachet pelable de stérilisation.
   ∘ Faciliter le gerbage de plusieurs systèmes emballés, en limitant la transmission de contraintes mécaniques inter systèmes.
- A l'étape de stérilisation, à la fois :
   ∘ Permettre la stérilisation intérieure du système.
   ∘ Eviter la transmission de contraintes mécaniques entre la poche et les tubulures et accessoires, lorsque la poche subit les éventuels gonflements au cours du cycle d'autoclavage (en raison de variations des pressions dans l'autoclave).

L'enjeu principal est la préservation de l'intégrité du système pendant l'autoclavage. Elle est d'autant plus difficile à préserver lorsque le système contient :
- Une poche souple potentiellement sujette à des cycles de gonflement / dégonflement,
- Des tubulures en PVC, dont la tenue mécanique décroît fortement à la température d'autoclavage (121°C),
- De nombreux accessoires plastiques injectés dont certains peuvent être relativement volumineux et contondants.

Il n'existe pas dans l'état de la technique de solution permettant de préserver cette intégrité, dans le cas de systèmes « poches, tubulures et accessoires » tels que décrits ci-dessus. Les systèmes existant dans l'état de la technique sont directement conditionnés « en vrac » dans un sachet pelable d'autoclavage, sans précaution particulière.

On connaît notamment dans l'état de la technique des poches à usage médical dans des emballages autoclavables composés d'une couche en film plastique transparent et d'une couche papier perméable à la vapeur d'eau. Parmi ces poches, certaines présentent des tubes en matériau PVC, mais généralement pas d'accessoires volumineux ou contondants.

On connaît également dans l'état de la technique des kits dans des emballages de type blister stérilisé par irradiation ou par oxyde-éthylène. La partie inférieure de ces emballages est généralement constituée d'une plaque de polymère thermoformée, l'opercule étant souple et pelable. Parmi ces kits, certains présentent des tubes en matériau PVC de différentes natures, et de nombreux accessoires rigides injectés, dont certains sont volumineux et/ou contondants.

US 2016/0015456 concerne un plateau chirurgical (« surgical tray 100 ») destiné à recevoir des instruments chirurgicaux (figures 12- 21) de manière provisoire pendant une opération chirurgicale.

FR2699146A1 décrit un emballage autoclavable pour un ensemble à usage unique de perfusion de fluide médical.

WO 2014/002938 A1 concerne un plateau destiné à recevoir une poche de sang et ses accessoires pour l'autoclavage.

US 2017/0290634 décrit un emballage (« package 50 ») pour un dispositif médical pour stérilisation par oxyde d'éthylène.

### Exposé de l'invention

La présente invention entend remédier aux inconvénients de l'art antérieur en proposant un plateau mis en forme, comportant une pluralité de logements, apte à recevoir une poche à usage médical, des tubulures et des accessoires, qui sont destinés à être stérilisés à la vapeur.

A cet effet, la présente invention concerne, dans son acception la plus générale, un plateau mis en forme, comportant une pluralité de logements, le plateau et chacun desdits logements étant aptes à recevoir une poche à usage médical, au moins une tubulure et au moins un accessoire, ladite poche à usage médical, ladite au moins une tubulure et ledit au moins un accessoire étant reliés entre eux, dans lequel ledit plateau est enveloppé dans un emballage pour effectuer un autoclavage.

Ainsi, le plateau selon la présente invention permet de :
- fournir un plan d'appui horizontal inférieur pour reposer à plat ;
- noyer les tubulures et accessoires dans des formes en dépression, les préservant de tout contact entre elles et avec la poche ;
- fournir un plan d'appui horizontal médian pour la poche, lui permettant de gonfler légèrement sans contrainte sur les tubes ou accessoires ;
- fournir un plan d'appui horizontal supérieur pour gerber plusieurs systèmes emballés (par exemple empilés « tête-bêche ») ;
- servir de gabarit, notamment pour vérifier, par contrôle visuel, que le système est correctement assemblé.

Selon un mode de réalisation, ledit plateau est constitué de polycarbonate.

Selon un mode de réalisation, ledit plateau comporte des découpes en son fond permettant un écoulement par gravité d'éventuels condensats.

Selon l'invention, ledit emballage comporte un film supérieur en matière plastique transparente, et un film inférieur en matière poreuse.

Ainsi, le film supérieur permet la visualisation du système en position dans le plateau, et le film inférieur permet la circulation de la vapeur et l'évacuation gravitaire des éventuels condensats.

Avantageusement, au moins un desdits logements est configuré pour permettre un déplacement de ladite au moins une tubulure dans l'axe de ladite au moins une tubulure.

De préférence, au moins un desdits logements est configuré pour empêcher un déplacement de ladite au moins une tubulure dans l'axe normal au plan dudit plateau.

Selon une variante, au moins un desdits logements comporte une butée bloquant ladite au moins une tubulure dans une position, selon un axe normal au plan dudit plateau.

Selon un mode de réalisation, au moins un desdits logements est configuré pour bloquer ledit au moins un accessoire dans une position prédéterminée.

Dans un mode de réalisation, ladite au moins une tubulure est libre en déplacement.

Selon un mode de réalisation, ledit plateau comporte des plans, tels que :
- un logement apte à recevoir ladite poche à usage médical se situe dans un premier plan ;
- deux autres logements aptes à recevoir ladite au moins une tubulure et ledit au moins un accessoire se situent respectivement dans un second plan et dans un troisième plan ;
ledit premier plan étant distinct desdits second et troisième plans et parallèle auxdits second et troisième plans.

Ceci permet d'augmenter la compacité du dispositif, tout en conservant l'intégrité du kit.

Selon un autre mode de réalisation, ladite poche à usage médical, ladite au moins une tubulure et ledit au moins un accessoire sont situés dans un même plan.

Avantageusement, ledit déplacement de ladite au moins une tubulure dans l'axe de ladite au moins une tubulure présente une longueur comprise entre 5 % et 25 % de la longueur de ladite au moins une tubulure.

De préférence, ledit déplacement de ladite au moins une tubulure dans l'axe de ladite au moins une tubulure présente une longueur comprise entre 10 % et 20 % de la longueur de ladite au moins une tubulure.

Selon une variante, ledit plateau est thermoformé.

Selon une autre variante, ledit plateau est réalisé par un procédé d'injection plastique.

Selon une autre variante, ledit plateau est obtenu par emboutissage.

Selon un mode de réalisation, ledit plateau est apte à être empilé sur un autre plateau du même type, tout en préservant l'intégrité de ladite poche à usage médical.

Selon un mode de réalisation, au moins un logement, apte à recevoir ladite poche à usage médical, est configuré pour permettre le gonflement de ladite poche à usage médical.

Selon un mode de mise en oeuvre particulier, ledit emballage est pelable.

### Brève description des dessins

On comprendra mieux l'invention à l'aide de la description, faite ci-après à titre purement explicatif, d'un mode de réalisation de l'invention, en référence aux Figures dans lesquelles :
- Les Figures 1, 2 et 3 illustrent le plateau selon la présente invention, dans un mode de réalisation ; et
- La Figure 4 représente un kit (poche à usage médical, tubulures et accessoires) selon la présente invention.

### Description détaillée de modes de réalisation de l'invention

La présente invention se rapporte à un plateau 10 mis en forme. Le plateau 10 selon la présente invention comporte une pluralité de logements 11, 12, 13, le plateau 10 et chacun desdits logements 11, 12, 13 étant aptes à recevoir une poche 20 à usage médical, au moins une tubulure 21 et au moins un accessoire 22, ladite poche 20 à usage médical, ladite au moins une tubulure 21 et ledit au moins un accessoire 22 étant reliés entre eux. De plus, ledit plateau 10 est enveloppé dans un emballage 30 pour effectuer un autoclavage.

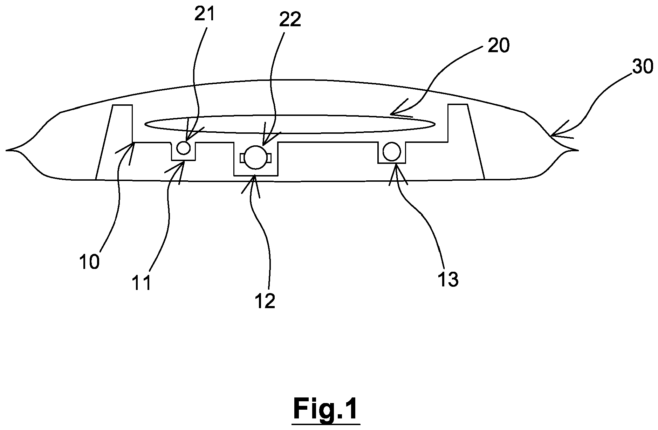

Les Figures 1, 2 et 3 illustrent le plateau selon la présente invention, dans un mode de réalisation.

On observe sur la Figure 1 le plateau 10 selon la présente invention, qui comporte une pluralité de logements 11, 12, 13. On observe également sur la Figure 1 une poche 20 à usage médical ainsi que des tubulures 21 et des accessoires 22, ces différents éléments étant reliés entre eux. Enfin, on observe sur la Figure 1 un emballage 30, qui enveloppe le plateau 10 selon la présente invention.

La poche et les tubulures sont relativement libres de mouvement sur les plans horizontaux, afin de leur permettre de se déformer sans résistance, notamment dans le cadre des retraits inévitables avec certains polymères aux températures évoquées.

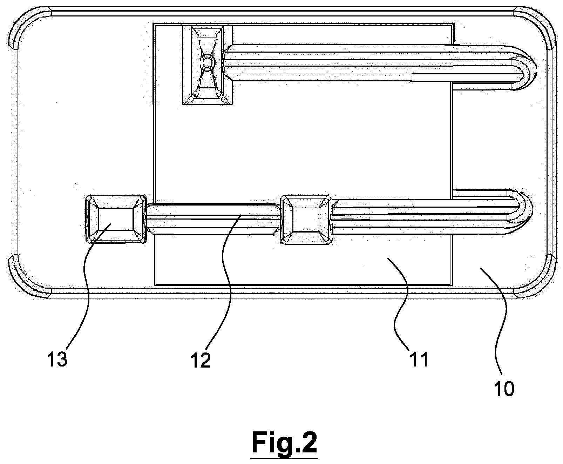

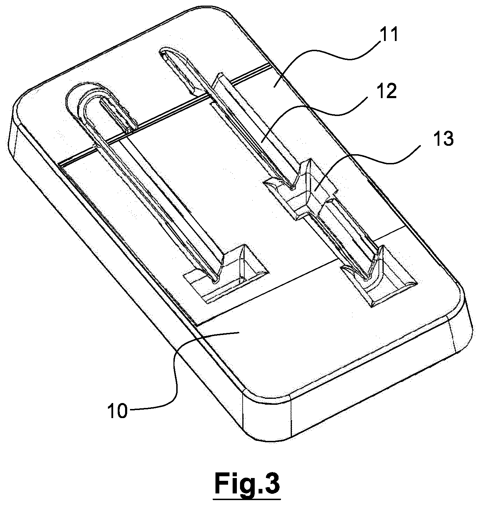

On observe également sur les Figures 2 et 3 le plateau 10 selon la présente invention, qui comporte une pluralité de logements 11, 12, 13.

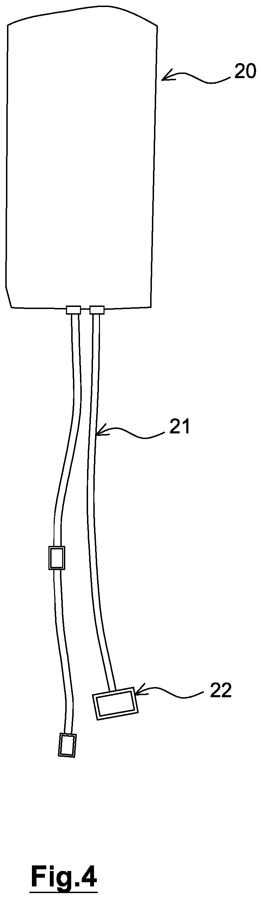

La Figure 4 représente un kit (poche à usage médical, tubulures et accessoires) selon la présente invention. Le kit illustré Figure 4 comporte une poche 20 à usage médical ainsi que des tubulures 21 et des accessoires 22, ces différents éléments étant reliés entre eux.

Selon l'invention, l'emballage 30 autoclavable est utilisé de manière à ce que le film supérieur soit en matière plastique transparente, permettant ainsi la visualisation du système en position dans le plateau, et de manière à ce que le film inférieur soit en matière poreuse, type papier, permettant la circulation de la vapeur et l'évacuation gravitaire des éventuels condensats.

Dans un mode de réalisation, le plateau 10 selon la présente invention est constitué de polycarbonate. Dans ce mode de réalisation, la présente invention est mise en oeuvre par le thermoformage d'une plaque en polycarbonate, qui est une matière supportant l'autoclavage à 121°C.

Dans un mode de réalisation, le plateau 10 selon la présente invention comporte des découpes en son fond permettant un écoulement par gravité d'éventuels condensats.

Dans un mode de réalisation, au moins un desdits logements 11, 12, 13 est configuré pour permettre un déplacement de ladite au moins une tubulure 21 dans l'axe de ladite au moins une tubulure 21.

Dans un mode de réalisation, au moins un desdits logements 11, 12, 13 est configuré pour empêcher un déplacement de ladite au moins une tubulure 21 dans l'axe normal au plan dudit plateau 10.

Dans un mode de réalisation, au moins un desdits logements 11, 12, 13 comporte une butée bloquant ladite au moins une tubulure 21 dans une position, selon un axe normal au plan dudit plateau 10.

Dans un mode de réalisation, au moins un desdits logements 11, 12, 13 est configuré pour bloquer ledit au moins un accessoire 22 dans une position prédéterminée.

Dans un mode de réalisation, ladite au moins une tubulure 21 est libre en déplacement.

Dans un mode de réalisation, le plateau 10 selon la présente invention comporte des plans P1, P2, P3, tels que :
- un logement 11 apte à recevoir ladite poche 20 à usage médical se situe dans un premier plan P1 ;
- deux autres logements 11, 12 aptes à recevoir ladite au moins une tubulure 21 et ledit au moins un accessoire 22 se situent respectivement dans un second plan P2 et dans un troisième plan P3 ;
et en ce que ledit premier plan P1 est distinct desdits second et troisième plans P2, P3 et parallèle auxdits second et troisième plans P2, P3.

Ceci permet d'augmenter la compacité du dispositif, tout en conservant l'intégrité du kit.

Dans un autre mode de réalisation, ladite poche 20 à usage médical, ladite au moins une tubulure 21 et ledit au moins un accessoire 22 sont situés dans un même plan.

Dans un mode de réalisation, ledit déplacement de ladite au moins une tubulure 21 dans l'axe de ladite au moins une tubulure 21 présente une longueur comprise entre 5 % et 25 % de la longueur de ladite au moins une tubulure 21.

Dans un mode de réalisation, ledit déplacement de ladite au moins une tubulure 21 dans l'axe de ladite au moins une tubulure 21 présente une longueur comprise entre 10 % et 20 % de la longueur de ladite au moins une tubulure 21.

Dans un mode de réalisation, le plateau 10 selon la présente invention est thermoformé.

Dans un autre mode de réalisation, le plateau 10 selon la présente invention est réalisé par un procédé d'injection plastique.

Dans un autre mode de réalisation, le plateau 10 selon la présente invention est obtenu par emboutissage.

Dans un mode de réalisation, le plateau 10 selon la présente invention est apte à être empilé sur un autre plateau du même type, tout en préservant l'intégrité de ladite poche 20 à usage médical.

Dans un mode de réalisation, au moins un logement 11, 12, 13, apte à recevoir ladite poche 20 à usage médical, est configuré pour permettre le gonflement de ladite poche 20 à usage médical.

Dans un mode de réalisation, ledit emballage 30 est pelable.

Le plateau 10 selon la présente invention trouve des applications dans les cas de stérilisation à la vapeur de systèmes creux composés de parties fragiles et déformables, et de parties rigides susceptibles de les marquer ou de les abîmer.

L'invention est décrite dans ce qui précède à titre d'exemple. Il est entendu que l'homme du métier est à même de réaliser différentes variantes de l'invention sans pour autant sortir du cadre du brevet, tel que défini par les revendications jointes.

## Revendications

1. Ensemble comportant :
- un système comprenant une poche (20) à usage médical, au moins une tubulure (21), et au moins un accessoire (22),
- un plateau (10) mis en forme, comportant une pluralité de logements (11, 12, 13), lesdits logements (11, 12, 13) étant aptes à recevoir respectivement la poche (20) à usage médical, la tubulure (21) et l'accessoire (22), ladite poche (20) à usage médical, ladite au moins une tubulure (21) et ledit au moins un accessoire (22) étant reliés entre eux, et
- un emballage (30) enveloppant le plateau (10) pour effectuer un autoclavage, l'emballage (30) comportant un film supérieur en matière plastique transparente, et un film inférieur en matière poreuse permettant une circulation de la vapeur.

2. Ensemble selon la revendication 1, dans lequel le plateau (10) est constitué de polycarbonate.

3. Ensemble selon l'une des revendications 1 ou 2, dans lequel le plateau (10) comporte des découpes en son fond permettant un écoulement par gravité d'éventuels condensats.

4. Ensemble selon l'une des revendications précédentes, dans lequel au moins un desdits logements (11, 12, 13) est configuré pour permettre un déplacement de ladite au moins une tubulure (21) dans l'axe de ladite au moins une tubulure (21).

5. Ensemble selon la revendication 4, **caractérisé en ce que** ledit déplacement de ladite au moins une tubulure (21) dans l'axe de ladite au moins une tubulure (21) présente une longueur comprise entre 5 % et 25 % de la longueur de ladite au moins une tubulure (21).

6. Ensemble selon la revendication 4, **caractérisé en ce que** ledit déplacement de ladite au moins une tubulure (21) dans l'axe de ladite au moins une tubulure (21) présente une longueur comprise entre 10 % et 20 % de la longueur de ladite au moins une tubulure (21).

7. Ensemble selon l'une des revendications précédentes, dans lequel au moins un desdits logements (11, 12, 13) est configuré pour empêcher un déplacement de ladite au moins une tubulure (21) dans l'axe normal au plan dudit plateau (10).

8. Ensemble selon l'une des revendications 4 à 7, dans lequel au moins un desdits logements (11, 12, 13) comporte une butée bloquant ladite au moins une tubulure (21) dans une position, selon un axe normal au plan dudit plateau (10).

9. Ensemble selon l'une des revendications 1 à 8, dans lequel au moins un desdits logements (11, 12, 13) est configuré pour bloquer ledit au moins un accessoire (22) dans une position prédéterminée.

10. Ensemble selon l'une des revendications précédentes, dans lequel le plateau (10) comporte des plans (P1, P2, P3), tels que :
- un logement (11) recevant ladite poche (20) à usage médical se situe dans un premier plan (P1) ;
- deux autres logements (11, 12) recevant ladite au moins une tubulure (21) et ledit au moins un accessoire (22) se situent respectivement dans un second plan (P2) et dans un troisième plan (P3) ;
et ledit premier plan (P1) est distinct desdits second et troisième plans (P2, P3) et parallèle auxdits second et troisième plans (P2, P3).

11. Ensemble selon l'une des revendications précédentes, dans lequel ladite poche (20) à usage médical, ladite au moins une tubulure (21) et ledit au moins un accessoire (22) sont situés dans un même plan.

12. Ensemble selon l'une des revendications 1 à 11, dans lequel le plateau (10) est thermoformé.

13. Ensemble selon l'une des revendications 1 à 11, dans lequel le plateau est réalisé par un procédé d'injection plastique.

14. Ensemble selon l'une des revendications 1 à 11, dans lequel le plateau (10) est obtenu par emboutissage.

15. Ensemble selon l'une des revendications 1 à 14 dans lequel ladite au moins une tubulure (21) est libre en déplacement.

16. Ensemble selon l'une des revendications 1 à 15 dans lequel ledit emballage (30) est pelable.

## Patentansprüche

1. Anordnung, aufweisend:
- ein System, das einen Beutel (20) für medizinische Zwecke, mindestens einen Schlauch (21) und mindestens ein Zubehör (22) umfasst,
- eine Formgebungsplatte (10), die eine Vielzahl von Aufnahmen (11, 12, 13) aufweist, wobei die Aufnahmen (11, 12, 13) imstande sind, jeweils den Beutel (20) für medizinische Zwecke, den Schlauch (21) und das Zubehör (22) aufzunehmen, wobei der Beutel (20) für medizinische Zwecke, der mindestens eine Schlauch (21) und das mindestens eine Zubehör (22) miteinander verbunden sind, und
- eine Verpackung (30), die die Platte (10) umhüllt, um eine Autoklavierung durchzuführen, wobei die Verpackung (30) eine obere Folie aus durchsichtigem Kunststoffmaterial und eine untere Folie aus porösem Material aufweist, das eine Dampfzirkulation gestattet.

2. Anordnung nach Anspruch 1, wobei die Platte (10) aus Polycarbonat besteht.

3. Anordnung nach einem der Ansprüche 1 oder 2, wobei die Platte (10) Ausschnitte in ihrem Boden aufweist, die ein Abfließen durch Schwerkraft von eventuellen Kondensaten gestatten.

4. Anordnung nach einem der vorhergehenden Ansprüche, wobei mindestens eine der Aufnahmen (11, 12, 13) ausgelegt ist, um eine Verlagerung des mindestens einen Schlauchs (21) in der Achse des mindestens einen Schlauchs (21) zu gestatten.

5. Anordnung nach Anspruch 4, **dadurch gekennzeichnet, dass** die Verlagerung des mindestens einen Schlauchs (21) in der Achse des mindestens einen Schlauchs (21) eine Länge zwischen 5% und 25% der Länge des mindestens einen Schlauchs (21) aufweist.

6. Anordnung nach Anspruch 4, **dadurch gekennzeichnet, dass** die Verlagerung des mindestens einen Schlauchs (21) in der Achse des mindestens einen Schlauchs (21) eine Länge zwischen 10% und 20% der Länge des mindestens einen Schlauchs (21) aufweist.

7. Anordnung nach einem der vorhergehenden Ansprüche, wobei mindestens eine der Aufnahmen (11, 12, 13) ausgelegt ist, um eine Verlagerung des mindestens einen Schlauchs (21) in der zur Ebene der Platte (10) normalen Achse zu verhindern.

8. Anordnung nach einem der Ansprüche 4 bis 7, wobei mindestens eine der Aufnahmen (11, 12, 13) einen Anschlag aufweist, der den mindestens einen Schlauch (21) in einer Position gemäß einer zur Ebene der Platte (10) normalen Achse blockiert.

9. Anordnung nach einem der Ansprüche 1 bis 8, wobei mindestens eine der Aufnahmen (11, 12, 13) ausgelegt ist, um das mindestens eine Zubehör (22) in einer vorbestimmten Position zu blockieren.

10. Anordnung nach einem der vorhergehenden Ansprüche, wobei die Platte (10) Ebenen (P1, P2, P3) aufweist, die derart sind, dass:
- sich eine Aufnahme (11), die den Beutel (20) für medizinische Zwecke aufnimmt, in einer ersten Ebene (P1) befindet;
- sich zwei weitere Aufnahmen (11, 12), die den mindestens einen Schlauch (21) und das mindestens eine Zubehör (22) aufnehmen, jeweils in einer zweiten Ebene (P2) und in einer dritten Ebene (P3) befinden;
wobei die erste Ebene (P1) von der zweiten und der dritten Ebene (P2, P3) verschieden und parallel zu der zweiten und der dritten Ebene (P2, P3) ist.

11. Anordnung nach einem der vorhergehenden Ansprüche, wobei sich der Beutel (20) für medizinische Zwecke, der mindestens eine Schlauch (21) und das mindestens eine Zubehör (22) in derselben Ebene befinden.

12. Anordnung nach einem der Ansprüche 1 bis 11, wobei die Platte (10) thermisch geformt ist.

13. Anordnung nach einem der Ansprüche 1 bis 11, wobei die Platte durch ein Kunststoffspritzverfahren hergestellt ist.

14. Anordnung nach einem der Ansprüche 1 bis 11, wobei die Platte (10) durch Tiefziehen hergestellt ist.

15. Anordnung nach einem der Ansprüche 1 bis 14, wobei der mindestens eine Schlauch (21) frei verlagerbar ist.

16. Anordnung nach einem der Ansprüche 1 bis 15, wobei die Verpackung (30) abziehbar ist.

## Claims

1. An assembly including:
- a system comprising a bag (20) for medical use, at least one tubing (21) and at least one accessory (22),
- a shaped tray (10), including a plurality of housings (11, 12, 13), said housings (11, 12, 13) being able to receive respectively the bag(20) for medical use, the tubing (21) and the accessory (22), said bag (20) for medical use, said at least one tubing (21) and said at least one accessory (22) being linked together, and
- a package (30) wrapping the tray (10) for autoclaving, the package (30) including an upper film made of transparent plastic material, and a lower film made of porous material, allowing circulation of steam.

2. The assembly according to claim 1, wherein the tray (10) is made of polycarbonate.

3. The assembly according to one of claims 1 or 2, wherein the tray (10) includes cutouts in its bottom allowing possible condensates to flow by gravity.

4. The assembly according to any of the preceding claims, wherein at least one of said housings (11, 12, 13) is configured to allow displacement of said at least one tubing (21) in the axis of said at least one tubing (21).

5. The assembly according to claim 4, **characterized in that** said displacement of said at least one tubing (21) in the axis of said at least one tubing (21) has a length comprised between 5% and 25% of the length of said at least one tubing (21).

6. The assembly according to claim 4, **characterized in that** said displacement of said at least one tubing (21) in the axis of said at least one tubing (21) has a length comprised between 10% and 20% of the length of said at least one tubing (21).

7. The assembly according to any of the preceding claims, wherein at least one of said housings (11, 12, 13) is configured to prevent displacement of said at least one tubing (21) in the axis normal to the plane of said tray (10).

8. The assembly according to any of claims 4 to 7, wherein at least one of said housings (11, 12, 13) includes an abutment blocking said at least one tubing (21) in one position, along an axis normal to the plane of said tray (10).

9. The assembly according to any of claims 1 to 8, wherein at least one of said housings (11, 12, 13) is configured to block said at least one accessory (22) in a predetermined position.

10. The assembly according to any of the preceding claims, wherein the tray (10) includes planes (P1, P2, P3), such as:
- a housing (11) receiving said bag (20) for medical use is located in a first plane (P1);
- two other housings (11, 12) receiving said at least one tubing (21) and said at least one accessory (22) are located respectively in a second plane (P2) and in a third plane (P3);
and said first plane (P1) is distinct from said second and third planes (P2, P3) and parallel to said second and third planes (P2, P3).

11. The assembly according to any of the preceding claims, wherein said bag (20) for medical use, said at least one tubing (21) and said at least one accessory (22) are located in the same plane.

12. The assembly according to any of claims 1 to 11, wherein the tray (10) is thermoformed.

13. The assembly according to any of claims 1 to 11, wherein the tray is produced by a plastic injection method.

14. The assembly according to any of claims 1 to 11, wherein the tray (10) is obtained by stamping.

15. The assembly according to any of claims 1 to 14, wherein said at least one tubing (21) is free in displacement.

16. The assembly according to any of claims 1 to 15, wherein said package (30) is peelable.
